(19) Europäisches Patentamt
European Patent Office
Office européen des brevets

(11) Veröffentlichungsnummer: **0 047 850**
**B1**

(12) # EUROPÄISCHE PATENTSCHRIFT

(45) Veröffentlichungstag der Patentschrift: **12.09.84**

(51) Int. Cl.[3]: **C 07 D 231/38,** A 01 N 43/56

(21) Anmeldenummer: **81105995.5**

(22) Anmeldetag: **30.07.81**

(54) **Verwendung von 4-Nitroso-Pyrazolen in mikrobiziden Mitteln.**

(30) Priorität: **09.08.80 DE 3030192**

(43) Veröffentlichungstag der Anmeldung: **24.03.82 Patentblatt 82/12**

(45) Bekanntmachung des Hinweises auf die Patenterteilung: **12.09.84 Patentblatt 84/37**

(84) Benannte Vertragsstaaten: **BE CH DE FR GB IT LI SE**

(56) Entgegenhaltungen:
**FR - A - 1 239 901**
**GB - A - 786 753**
**GB - A - 791 688**
**US - A - 2 510 724**
**US - A - 2 751 395**
**US - A - 2 831 866**

**JOURNAL OF ORGANIC CHEMISTRY, Band 38, Nr. 17, 1973, Seiten 2958-2963 Easton, U.S.A., P.A.S. SMITH et al.: "Thermally induced fragmentation of some azidopyrazole derivatives"**
**CHEMICAL ABSTRACTS, Band 44, Nr. 1, 10. Januar 1950, Columbus, Ohio, U.S.A. G.L. McNEW et al.: "Fungicidal activity of substituted pyrazoles and related compounds"**
**CHEMICAL ABSTRACTS, Band 49, Nr. 1, 10. Januar**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Riebel, Hans-Jochem, Dr., In der Beek 92, D-5600 Wuppertal 1 (DE)**
Erfinder: **Paulus, Wilfried, Dr., Deswatinesstrasse 90, D-4150 Krefeld 1 (DE)**
Erfinder: **Genth, Hermann, Dr., Am Heckerhof 60, D-4150 Krefeld 1 (DE)**

(56) Entgegenhaltungen: (Fortsetzung)
**1955, Columbus, Ohio, U.S.A. E.L. FRICK: "Laboratory tests of the effects of fungicides on the sporegermination of Botrytis cinerea"**
**CHEMICAL ABSTRACTS, Band 51, Nr. 1. 10. Januar 1957, Columbus, Ohio, U.S.A. HIROKUNI TAMURA: "Evaluation of organic fungicides. Toxicity of several substituted pyrazoles"**
**CHEMICAL ABSTRACTS, Band 60, Nr. 1, 6. Januar 1964, Columbus, Ohio, U.S.A. V.G. ERMOLAEVA et al.: "Pyridylthiazolylmethane series. III. Synthesis and properties of 3-pyridyl-2'-thiazolylcarbinols"**

**Die Akte enthält technische Angaben, die nach dem Eingang der Anmeldung eingereicht wurden und die nicht in dieser Patentschrift enthalten sind.**

EP 0 047 850 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von 4-Nitroso-pyrazolen in mikrobiziden Mitteln zum Schutz technischer Materialien.

Es ist bekannt, dass bestimmte 4-Nitroso-pyrazole, wie z.B. das 3,5-Dimethyl-1-iso-propyl-4-nitroso-pyrazol, das 3,5-Dimethyl-1-(4-chlor-phenyl)-4-nitroso-pyrazol und das 3,5-Dimethyl-1-(4-methyl-phenyl)-4-nitroso-pyrazol fungizide und zum Teil auch bakterizide Eigenschaften aufweisen und in Pflanzenschutzmitteln verwendet werden können (GB 786753, US 2510724 und Phytopathology 39, 721–751 (1949).

Aus CA 60 (1964) 514a–515c ist 1-Benzyl-3,5-dimethyl-4-nitroso-pyrazol bekannt.

Aus der US 2831866 ist bekannt, dass Nitrosopyrazole im wesentlichen zur Bekämpfung pathogener Pilze bei Menschen und Tieren geeignet sind.

Es wurde die Verwendung von 4-Nitroso-pyrazolen der Formel

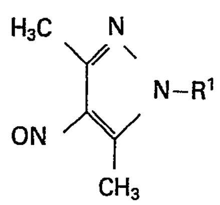

(I)

worin

$R^1$ für Benzyl, welches gegebenenfalls durch Halogen, Nitro, $C_1$ bis $C_4$-Alkyl und/oder $C_1$ bis $C_4$-Alkoxy substituiert ist, steht,

in mikrobiziden Mitteln zum Schutz technischer Materialien gefunden.

Überraschenderweise zeigen die erfindungsgemäss zu verwendenden mikrobiziden Mittel zum Schutz technischer Materialien entsprechend der Formel (I) eine erhebliche höhere fungizide und bakterizide Wirkung als bekannte Verbindungen analoger Konstitution und gleicher Wirkungsrichtung.

Alkyl- und Alkoxyreste enthalten einen geradkettigen oder verzweigten Kohlenwasserstoffrest mit 1 bis 4 Kohlenstoffatomen. Beispielsweise seien die folgenden Alkyl- und Alkoxyreste genannt: Methyl, Ethyl, Propyl, Isopropyl, Butyl und Isobutyl und Methoxy, Ethoxy, Propoxy, Isopropoxy, Butoxy und Isobutoxy. Bevorzugte Alkyl- und Alkoxyreste sind der Methyl- und Ethylrest und der Methoxy- und Ethoxyrest.

Die 4-Nitroso-pyrazole können hergestellt werden, indem man Hydrazinderivate der Formel

$$H_2N-NH-R^1$$

worin

$R^1$ die oben angegebene Bedeutung hat

oder deren Salze mit Isonitroso-acetylaceton in wässriger Dispersion bei Temperaturen zwischen –20 und 50°C umsetzt.

Die Umsetzung kann durch die folgende Reaktionsgleichung erläutert werden:

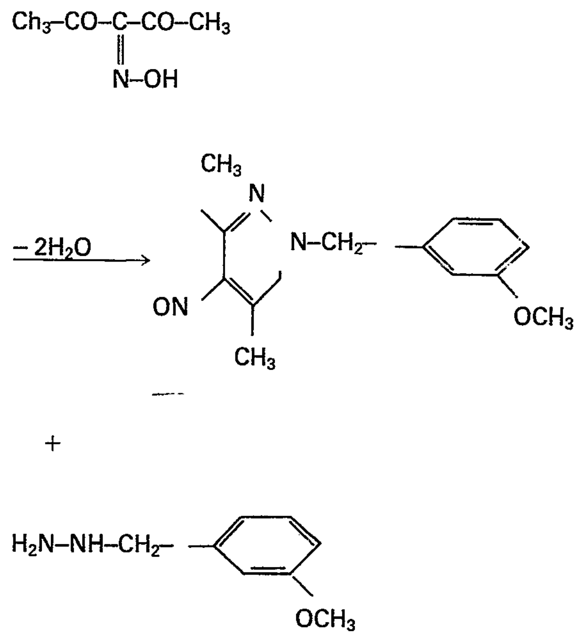


Die Hydrazinderivate sind an sich bekannt (Methodicum Chimicum, Band 6, S. 75–131, Georg Thieme Verlag, Stuttgart 1974) und können nach bekannten Verfahren, beispielsweise durch Umsetzung von Hydrazin mit Acrylnitril, von Diazoverbindungen mit Bisulfiten, Nitrosoverbindungen mit Reduktionsmitteln oder von Halogenaromaten mit Hyrazin hergestellt werden. Es ist auch möglich, anstelle der Hydrazinderivate die Vorprodukte, wie z.B. Hydrazin und Acrylnitril ohne Zwischenisolierung des Hydrazinderivats zur Herstellung des 4-Nitroso-pyrazols einzusetzen.

Die Salze der Hydrazinderivate können durch Umsetzung des Hydrazinderivats mit einer Säure, beispielsweise einer Mineralsäure wie Salz- oder Schwefelsäure, hergestellt werden.

Das als weitere Ausgangskomponente zu verwendende Isonitroso-acetylaceton ist ebenfalls an sich bekannt (Wolff, Liebigs Annalen der Chemie, 325, 139) und kann aus Acetylaceton und einem Nitrit in Gegenwart einer Säure hergestellt werden.

Bei der Herstellung der 4-Nitroso-pyrazole kann die Reaktionstemperatur innerhalb eines grösseren Bereiches variiert werden. Im allgemeinen arbeitet man im Temperaturbereich von –20 bis 50°C, vorzugsweise im Temperaturbereich von 0 bis 30°C. Das Verfahren wird im allgemeinen bei Normaldruck durchgeführt.

Die Ausgangsverbindungen werden im allgemeinen in annähernd äquimolaren Mengen verwendet. Je Mol Hydrazinderivat setzt man zwischen 0,5 und 1,5, vorzugsweise zwischen 0,8 und 1,2 Mol, Isonitroso-acetylaceton ein.

Das erfindungsgemässe Verfahren kann beispielsweise wie folgt durchgeführt werden:

Das Hydrazinderivat wird in Wasser gelöst und mit Eis gekühlt. Zu dieser Lösung gibt man eine wässrige Lösung von Isonitrosoacetylaceton tropfenweise hinzu.

Die Reaktionsmischung wird 1 bis 3 Stunden gerührt. Soweit die Produkte hierbei kristallin anfallen, werden sie durch Absaugen isoliert. Anderenfalls kann die Aufarbeitung nach üblichen Methoden erfolgen, beispielsweise durch Extraktion mit einem mit Wasser praktisch nicht mischbaren organischen Lösungsmittel, wie z.B. Diethylether, Abtrennen der organischen Extrakte und Eindampfen dieser Lösungen. Die Produkte können gegebenenfalls auch umkristallisiert werden.

Technische Materialien, die durch die erfindungsgemässen 4-Nitroso-pyrazole vor einer mikrobiellen Veränderung und Zerstörung geschützt werden sollen, sind beispielsweise Klebstoffe, Leime, Papiere und Kartone, Textilien, Leder, Holz, Anstrichmittel, Putze, Kühlschmiermittel und Kunststoffartikel, die von Mikroorganismen befallen und zersetzt werden können. Im Rahmen der zu schützenden Materialien seien auch Teile von Produktionsanlagen, wie beispielsweise Kühlwasser- und Kühlschmiermittelkreisläufe, genannt, deren Funktionstüchtigkeit durch Mikroorganismen beeinträchtigt werden kann.

Mikroorganismen, die einen Abbau oder eine Veränderung der technischen Materialien bewirken können, sind beispielsweise Bakterien, Pilze, Hefen, Algen, Schleime und Viren. Vorzugsweise wirken die erfindungsgemässen 4-Nitroso-pyrazole gegen Bakterien und Pilze.

Es seien beispielsweise Mikroorganismen der folgenden Gattungen genannt:
Escherichia, wie Escherichia coli,
Pseudomonas, wie Pseudomonas aeruginosa,
Staphylococcus, wie Staphylococcus aureus,
Alternaria, wie Alternaria tenuis,
Aspergillus, wie Aspergillus niger,
Chaetomonium, wie Chaetomonium globosum,
Coniophora, wie Coniophora cerebella,
Lentinus, wie Lentinus tigrinus,
Penicillium, wie Penicillium glaucum,
Polyporus, wie Polyporus versicolor,
Pullularia, wie Pullularia pullulans,
Sclerophoma, wie Sclerophoma pityophila,
Trichoderma, wie Trichoderma viride.

Je nach ihrem Anwendungsgebiet können die erfindungsgemässen 4-Nitroso-pyrazole in die üblichen Formulierungen überführt werden, wie Lösungen, Emulsionen, Suspensionen, Pulver, Pasten und Granulate. Diese können in an sich bekannter Weise hergestellt werden, z.B. durch Vermischen der Wirkstoffe mit einem Streckmittel, mit flüssigen Lösungsmitteln und/oder festen Trägerstoffen, gegebenenfalls unter Verwendung von Obeflächenaktiven Mitteln, wie Emulgatoren und/oder Dispergiermitteln, wobei beispielsweise im Falle der Benutzung von Wasserstreckmitteln, gegebenenfalls organische Lösungsmittel, als Hilfslösungsmittel verwendet werden können.

Organische Lösungsmittel für die Wirkstoffe können beispielsweise Alkohole, wie niedere aliphatische Alkohole, vorzugsweise Ethanol oder Isopropanol, oder Benzylalkohol, Ketone wie Aceton oder Methylethylketon, flüssige Kohlenwasserstoffe, wie Benzinfraktionen, chlorierte Kohlenwasserstoffe, wie 1,2-Dichlorethan sein.

Die Anwendungskonzentration der erfindungsgemässen 4-Nitroso-pyrazole richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 0,5 Gew.-%, bezogen auf das zu schützende Material.

Die erfindungsgemässen neuen Wirkstoffe können auch in Mischung mit anderen bekannten Wirkstoffen vorliegen. Beispielsweise seien die folgenden Wirkstoffe genannt: Benzimidazolylmethylcarbamat, Tetramethyl-thiuramdisulfid, N-Fluordichlormethylthio-phthalimid und N,N-Dimethyl-N'-phenyl-(N'-fluordichlormethylthio)-sulfamid, Formaldehyd abspaltende Verbindungen, wie Hemiformale, Oxazolidine, Hxahydro-s-triazine, N-Methylolamide und Phenolderivate, wie p-Chlor-m-Kresol, 2-Phenyl-phenol, (2,2'-Dihydroxy-5,5'-dichlor)-diphenylmethan.

A) Herstellungsbeispiele

Beispiel 1

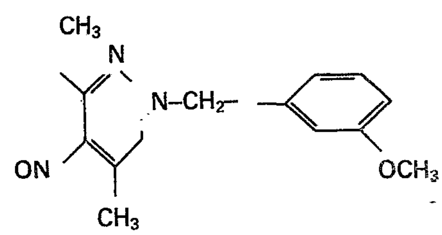

Zu einer Lösung von 19,5 g (0,1 Mol) Benzylhydrazin-dihydrochlorid in 200 ml Wasser werden bei 0–5°C unter heftigem Rühren 12,9 g (0,1 Mol) Isonitrosoacethylaceton in 100 ml Wasser getropft. Man rührt 2 Stunden bei Zimmertemperatur nach, extrahiert das Reaktionsgemisch zweimal mit je 100 ml Ether und dampft die vereinigten Etherextrakte dann ein. Man erhält 18 g (84% der Theorie) 3,5-Dimethyl-1-benzyl-4-nitroso-pyrazol in Form blaugrüner Kristalle.

Nach dem Umkristallisieren aus Benzol/Ligroin erhält man die reine Substanz mit einem Schmelzpunkt von 64°C.

Beispiel 2

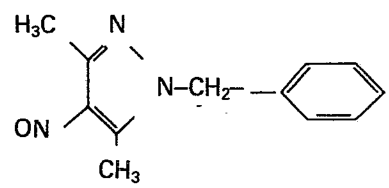

Zu einer heftigen gerührten Lösung von 12,9 g (0,1 Mol) Isonitrosoacetylaceton in 100 ml Wasser tropft man bei 0°C innerhalb von 2 Stunden eine wässrige Lösung von Benzylhydrazin (hergestellt aus 19,4 g (0,1 Mol) Benzylhydrazin-dihydrochlorid, 150 ml Wasser und 16,8 g (0,2 Mol) Natriumhydrogencarbonat). Nachdem ca. 1/10 der Benzylhydrazinlösung zugegeben ist, werden einige Impfkristalle von 3,5-Dimethyl-1-benzyl-4-nitroso-pyrazol zugesetzt. Es wird 5 Stunden bei einer Temperatur von 0–20°C nachgerührt. Dann wird der blaue, kristalline Niederschlag abgesaugt, mit kaltem Wasser gewaschen und an der Luft getrocknet.

Man erhält 20 g (93% der Theorie) 3,5-Dimethyl-1-benzyl-4-nitroso-pyrazol in Form blauer Kristalle vom Schmelzpunkt 64%.

B) Anwendungsbeispiele

Beispiel 3

Zum Nachweis der Wirksamkeit gegen Pilze werden die minimalen Hemm-Konzentrationen (MHK) von erfindungsgemässen Wirkstoffen bestimmt:

Ein Agar, der aus Bierwürze und Pepton hergestellt wird, wird mit erfindungsgemässen Wirkstoffen in Konzentrationen von 0,5 mg/l bis 5000 mg/l versetzt. Nach Erstarren des Agars erfolgt Kontamination mit Reinkulturen der in der Tabelle aufgeführten Testorganismen. Nach zweiwöchiger Lagerung bei 28°C und 60 bis 70% relativer Luftfeuchtigkeit wird die MHK bestimmt. MHK ist die niedrigste Konzentration an Wirkstoff bei der keinerlei Bewuchs durch die verwendete Mikrobenart erfolgt, sie ist in der nachstehenden Tabelle angegeben.

Tabelle I: Angabe der MHK-Werte in mg/l bei der Einwirkung der unten angegebenen Wirkstoffe auf Pilze

Wirkstoff: 3,5-Dimethyl-1-benzyl-4-nitroso-pyrazol

| Testpilze | |
|---|---|
| Aspergillus niger | 5 |
| Aspergillus terreus | 20,0 |
| Chaetomium globosum | 2,0 |
| Penicillium glaucum | 2,0 |
| Pullulaira pullulans | <1,0 |
| Rhizopus nigricans | 2,0 |

Beispiel 4

(Konservierung eines Kühlschmiermittels)

Ein Kühlschmierstoff auf Mineralölbasis wird mit 0,2% 3,5-Dimethyl-1-benzyl-4-nitroso-pyrazol versetzt und dann auf 5% mit Wasser verdünnt, so dass die Gebrauchsverdünnung 0,01% erfindungsgemässen Wirkstoff enthält.

Während 12 Wochen wird die Gebrauchsverdünnung täglich massiv mit Mikroben (Bakterien und Schimmelpilze), die aus einem mikrobiell verdorbenen Kühlschmiermittel stammen, kontaminiert. Zur Kontrolle der konservierenden Wirkung des Mikrobizids wird jeweils 24 Stunden nach einer Kontamination die Keimzahl bestimmt.

Ergebnis: Auch nach 12 Wochen ist das konservierte Kühlschmiermittel noch keimfrei; hingegen enthält ein nicht konserviertes Kühlschmiermittel, das in gleicher Weise behandelt wurde, bereits nach der ersten Kontamination ca. $10^7$ Keime/ml und zeigt nach 12 Wochen deutliche Zeichen mikrobieller Zusammensetzung.

**Patentanspruch:**

1. Verwendung von 4-Nitroso-pyrazolen der Formel

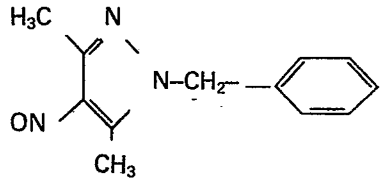

worin

$R^1$ für Benzyl, welches gegebenenfalls durch Halogen, Nitro, $C_1$- bis $C_4$-Alkyl und/oder $C_1$- bis $C_4$-Alkoxy substituiert ist, steht,

in mikrobiziden Mitteln zum Schutz technischer Materialien.

**Claims**

1. Use of 4-nitroso-pyrazoles of the formula

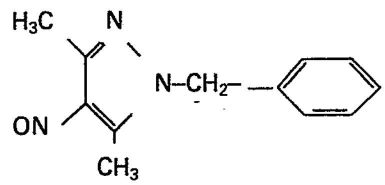

wherein

$R^1$ represents benyzl which is optionally substituted by halogen, nitro, $C_1$- to $C_4$-alkyl and/or $C_1$- to $C_4$-alkoxy,

in microbicidal agents for protecting industrial materials.

**Revendication**

1. Utilisation de 4-nitroso-pyrazoles de formule

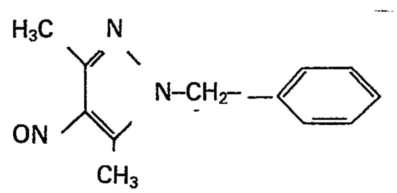

dans laquelle

$R^1$ représente un groupe benzyle qui est éventuellement substitué par halogène, nitro, alkyle en $C_1$–$C_4$ et/ou alcoxy en $C_1$–$C_4$,

dans des agents microbicides pour la protection de matériaux industriels.